# EUROPEAN PATENT APPLICATION

(11) **EP 1 174 440 A1**
(43) Date of publication of application: **23.01.2002**
(21) Application number: 00202597.1
(22) Date of filing: 19.07.2000
(51) Int. Cl.: C07K 16/28, G01N 33/577, G01N 33/569, A61K 39/395, C12N 15/13, C12N 1/21, A61P 31/00, A61P 35/00, A61P 37/00

(54) **A selectively-expressed epitope on the human CD38 molecule detected by a phage display library-derived human scFv antibody fragment**

(71) Applicant: U-BiSys B.V., 3584 CX Utrecht (NL)
(72) Inventor: Logtenberg, Ton, 3433 CH Nieuwegein (NL); Cilenti, Lucia, 00183 Roma (IT)
(74) Representative: Prins, Adrianus Willem

(57) **Abstract**

The present invention relates to antibodies and other binding molecules comprising a specificity for a selectively expressed CD38 epitope. Using a binding molecule of the invention it is, among others, possible to selectively mark a subset of CD38 expressing cells. An antibody or binding molecule of the invention is capable of binding to malignant B cells in a host thereby at least in part eliminating such malignant B cells from the body of the host. The invention among others provides pharmaceutical compositions, binding molecules, nucleic acids encoding binding molecules of the invention and gene delivery vehicles comprising said nucleic acid.

## Description

The invention relates to the field of medicine. The invention in particular relates to the diagnostic and/or medical use of antibodies.

Antibodies and binding molecules, in general, are widely used for the detection of biological substances. They are particularly popular for use in diagnostic methods. In addition, antibodies have enormous potential in human therapy. Because of their high binding specificity, antibodies are thought to be capable of binding to viruses and bacteria and their toxic products, facilitating their elimination. In other applications, antibodies were envisaged to specifically bind to tumor cells to promote their eradication or to bind to soluble molecules produced by cells of the immune system to neutralize their activity in harmful chronic inflammatory conditions and/or in autoimmune disease. Indeed, monoclonal antibodies have been described as magic bullets that could be used in the treatment of a wide variety of human diseases (1).

Monoclonal antibodies (MAbs) directed against cell surface antigens have played a pivotal role in studies of the phenotypic changes associated with cellular activation and differentiation. Conventionally, MAbs are generated by immortalization of the B lymphocytes of mice immunized with the antigen of interest and screening of hybridoma culture supernatant for the desired antibody specificities. More recently, the construction of large libraries of filamentous bacteriophage particles expressing antibody fragments and the development of various phage selection strategies has provided an alternative to hybridoma technology (2,3). In an approach recently developed by us, panning on eukaryotic cells in combination with a flow cytometry, may be employed to obtain phage antibodies specific against surface antigens (4). This phage selection strategy appears particularly useful when semi-synthetic phage antibody libraries, constructed *in vitro* from antibody variable gene segments and synthetic complementarity determining regions are used (5).

We and others have previously argued that with such libraries, the diversity and specificity of antibodies that can be obtained by phage panning on antigens are independent of the regulatory mechanisms that govern natural immune responses. Rather, the size and quality of the library, in combination with the selection procedure of choice and factors related to recombinant protein expression and propagation of filamentous phages affect the diversity and specificity of the antibodies that can be obtained (4-7).

In the present invention we have used a synthetic library of human scFv antibody fragments expressed on phage particles, in combination with cell sorting to select scFv antibodies that bind to the malignant plasma cells derived from the bone marrow of patients with multiple myeloma. Multiple myeloma is one example of debilitating plasma cell disorders for which as yet no satisfactory cure is available. It has been suggested to develop an antibody-based immunotherapy for such diseases. Candidate antigens that have been targeted or proposed for targeting include CD19, CD38, CD54, CD126, HM1.24 and Muc-1 core protein. Unfortunately, many of these antigens are not ideal for use in such immunotherapy since they are selectively expressed, are either shed or secreted, or have not been fully characterized. In the present invention, a stroma cell-dependent plasma cell line, established from the bone marrow (BM) of a MM patient was used as a target for phage selections. One of the monoclonal phage antibodies (mφAbs), named UM16, was further characterized and shown to bind to an epitope on the CD38 molecule, a type II integral membrane protein with a broad tissue and cellular distribution pattern. The human CD38 molecule, a 46 kD glycoprotein, is expressed on a broad variety of cell lineages (reviewed in 8). In the hematopoietic system, CD38 is expressed by plasma cells, monocytes, myeloid cells, T and B lymphocytes, NK cells, follicular dendritic cells and platelets (reviewed in 9-11). Strikingly, the UM-16 epitope was exclusively expressed on malignant and normal plasma cells and on discrete populations of *in vivo* activated B-and T lymphoid cells. Further analysis suggested that the UM16 epitope is an epitope expressed only upon activation of lymphoid cells but not of CD38+ cells of other lineages.
The epitope recognized by scFv UM16 lacks the broad cellular and tissue distribution of other reported CD38 epitopes. ScFv UM16 is expressed, among others, on normal and malignant plasma cells and defines an activation epitope on B and T lymphoid cells.

The present invention demonstrates that the CD38 protein comprises selectively expressed epitopes that can function as targets for binding moieties, novel methods are provided for the use of antibodies directed against the CD38 protein in a diagnostic and therapeutic setting.

The invention therefore provides a binding molecule capable of selectively binding to a subset of CD38 proteins. It is clear that a person skilled in the art will now be able to find other binding molecules capable of selectively binding to a subset of CD38 proteins. Furthermore, now that the present invention revealed the favorable features and uses of a selectively expressed CD38 epitope, a person skilled in the art will have the incentive to determine such selectively expressed CD38 epitopes.
In the present invention it has been found that such a binding molecule can be used to selectively mark a subset of CD38 expressing cells. A binding molecule with such properties can favorably be used for selecting a subset of CD38 protein expressing cells. The binding molecule can be used for diagnostic purposes to determine, type and or quantify in a sample, the number of cells displaying the selectively expressed CD38 epitope. In a therapeutic setting this property can be used, for instance, to selectively mark the subset of cells with the binding molecule without essentially marking other cells.

Preferably, the binding molecule is capable of binding to a part of the CD38 protein that is present on the outwardly directed side of the cell membrane of the subset of cells. This enables the easy detection of the subset of cells when the cells are essentially intact.

A selectively expressed CD38 epitope may be entirely absent on a group of CD38 positive cells not capable of being recognized by the binding molecule. However, this in not essential. The selectively expressed epitope, however, should not be available for binding to the binding moiety on such a group of cells. Availability of the epitope for binding of the binding molecule can be absent on such a group of cells for a number of different reasons. For example, the epitope can be absent from CD38 expressed by the group of cells. However, availability can also be lacking, due to a strongly reduced access of the CD38 binding molecule to the epitope. Reduced access can, for instance, be due to the binding of one or more other molecules at or near the epitope. Reduced access can also be the result of a particular spatial organization of the epitope. For instance the epitope may be present in a fold of the CD38 protein that is not accessible for the binding molecule, or may alternatively be present on a compartment that is not accessible for the binding molecule, such as on the other side of a plasma cell membrane.

In a preferred embodiment of the invention, the subset of cells comprises a plasma cell and/or a derivative thereof. Plasma cells are B-cells actively producing large amounts of antibody.

During early human B cell differentiation in fetal BM, UM16-expression correlated with the activation status of the developing B cells (12). The UM16 epitope was detectable on the earliest identifiable pro-B cells and continued to be present during all stages of human fetal B cell differentiation, up to the mature sIgM+ B cells. In the peripheral blood compartment, dominated by resting B cells, no UM16+ B cells could be detected. In fact, no UM16+ cells could be detected in blood mononuclear cells, including populations of CD38+ monocytes and NK cells.

In human tonsils, scFv UM16-stained activated B-lineage cells with a germinal center founder, germinal center and plasma blast phenotype, whereas non-activated, virgin and memory B cells were uniformly UM16 negative. A subpopulation of UM16+ T cells expressing high levels of CD38 could only be detected in some tonsils. In other lymphoid organs UM16+ T cells were not detectable.

Data demonstrated that the lymphoid restricted expression pattern of UM16 could not be simply attributed to a lower affinity of the scFv UM16 compared to conventional murine monoclonal antibodies. In a panel of cell lines expressing comparably high levels of CD38, some cell lines completely lacked scFv UM16 reactivity whereas others clearly exposed the UM16 epitope. Similarly, in blood, CD38+ mononuclear cells were detectable yet lacked the UM16 epitope. Among *in vivo* activated tonsillar T cells that uniformly expressed high levels of CD38, a subpopulation expressed UM16+. Thus, in a preferred embodiment, said subset of cells comprises an activated hemopoietic cell. Preferably,. activation of said hemopoietic cell occurs *in vivo.* Activation of T-cells and/or B-cells is correlated with the expression of a selective CD38 phenotype on the surface of at least a subset of the activated T-cells and/or B-cells. Activation of T-cells and/or B-cells is correlated with activation and/or activity of an immune response. The ability to target such cells with a binding molecule in a body allows one to selectively kill this subset of T and or B cells without essentially killing resting B and T cells in a body.

A binding molecule of the invention can be any kind of molecule capable of selectively binding to a subset of CD38 proteins. Preferably, said molecule comprises a protein. In one embodiment the binding molecule of the invention is selected from phage antibody display libraries. Phage antibody display libraries are large collections of antibody fragments expressed on bacteriophage particles. Phage antibody display libraries are constructed in vitro and mimmick key features of the humoral immune response (3,13). For the construction of phage display libraries, collections of human antibody heavy and light chain variable region genes are expressed on the surface of bacteriophage particles, either in single chain Fv (scFv) or in Fab format. Large libraries of antibody fragment-expressing phages typically contain > 10⁹ antibody specificities and may be assembled from the immunoglobulin V regions expressed in the B lymphocytes of immunized or non-immunized individuals. Alternatively, phage display libraries may be constructed from immunoglobulin variable regions that have been partially assembled in vitro to introduce additional antibody diversity in the library ('semi-synthetic' libraries). For example, in vitro assembled variable regions contain stretches of synthetically-produced, randomized or partially-randomized DNA in those regions of the molecules that are important for antibody specificity (5,14). Thus, specificities not present in natural repertoires may be added to phage display libraries of antibody fragments.

Recombinant phages expressing antibody fragments of desirable specificities may be selected from a library by one of several methods. Target antigens are immobilized on a solid phase and subsequently exposed to a phage library to allow binding of phages expressing antibody fragments specific for the solid phase-bound antigen. Non-bound phages are removed by washing and bound phages eluted from the solid phase for infection of Escherichia Coli bacteria and subsequent propagation. Multiple rounds of selection and propagation are usually required to sufficiently enrich for phages binding specifically to the target antigen. Phages may also be selected for binding to complex antigens such as complex mixtures of proteins or whole cells. Selection of antibodies on whole cells advantageously permits presentation of target antigens in their native configuration, unperturbed by conformational changes that are introduced by immobilizing an antigen to a solid phase.

The constraints imposed by the natural immune response and the influence of the immunogenicity of the target antigen do not permit the isolation of monoclonal antibodies against any antigen by conventional hybridoma technology. In phage approaches, these factors do not play a role, allowing the isolation of monoclonal antibodies against "difficult" antigens such as autoantigens, carbohydrates and toxic antigens. Considering that CD38 proteins from different species often comprise substantial homology, it is preferred to select the binding molecule of the invention from an *in vitro* selection system for binding molecules. Preferably, the binding molecule comprises a CD38 binding part of a single chain antibody. A binding part of a single chain antibody or a Fab fragment typically comprises at least part of the immunoglobulin V regions.

Numerous clinical studies with monoclonal antibodies of non-human (usually murine) origin performed poorly as a result of their immunogenicity. Upon injection of murine antibodies in humans, the human immune system recognizes the murine antibodies as foreign proteins, resulting in the induction of an immune response against the murine protein (15,16). In addition, murine antibodies have poor pharmacokinetic properties in humans (17,18) and are inefficient in recruiting effector functions of cells of the immune system (19,20). These issues have spurred the development of alternative strategies to obtain more human antibodies for therapy (reviewed in 21).

In one embodiment for creating more human antibodies, the immunoglobulin variable regions encoding murine antibodies are genetically used to human immunoglobulin constant regions. The resulting chimeric antibody contains greater than 30% murine amino acid sequences. Clinical application in humans of chimeric monoclonal antibodies, has shown that these proteins retain their immunogenicity in the majority of cases (22,23). In another embodiment, only immunoglobulin variable region sequences relevant for antibody specificity are of murine origin are used. The constant regions of the immunoglobulin molecule, as well as the framework regions of the variable region, are of human origin. Clinical application of these "humanized" monoclonal antibodies indicates that these molecules are generally more effective and have no or little intrinsic toxicity or immunogenicity (17,24). The binding molecule of the invention therefore preferably comprises a humanized antibody and/or humanized Fab-fragment, or a functional part, derivative and/or analogue thereof. A functional part, derivative and/or analogue of a humanized antibody and/or humanized Fab-fragment of the invention exhibits the same CD38 binding activity in quality, but not necessarily in quantity. They further exhibit an essentially similar low immune-response inducing capacity and/or similar favorable pharmacokinetics in a human body.
Reconstructing the original affinity and specificity in a humanized version of a murine antibody is a time-consuming process that may render the antibody less human (25). These considerations with chimeric and humanized monoclonal antibodies and the recent clinical success of engineered monoclonal antibodies spurred the development of efficient methods for the isolation and production of fully human monoclonal antibodies, the most desirable antibody format for clinical application.

One method of obtaining human monoclonal antibodies employs transgenic mice harboring human immunoglobulin loci in combination with conventional hybridoma technology (26-28). In these mice, large portions of human immunoglobulin heavy and light chain loci have been inserted in the mouse germ line while the endogenous murine immunoglobulin loci have been silenced by gene knockout. Immunization of these transgenic mice with an antigen results in the production of human antibodies specific for the antigen. Human monoclonal antibody-producing cell lines can be obtained from these mice by fusing the spleen cells of immunized mice with plasma cell lines in vitro to obtain immortalized monoclonal antibody-secreting hybridomas. Importantly, production of human antibodies in transgenic mice depends on immunization procedures and is governed by constraints of the murine immune response. As a consequence, it is difficult, if not impossible, to obtain antibodies against the mouse's own antigens ("autoantigens"), to xenoantigens that have a high degree of homology to murine autoantigens or to antigens that have poor immunogenic properties, such as polysaccharides. These notions have spurred the development of molecular approaches that obviate the need for immunization and cell immortalization to obtain human monoclonal antibodies with desired specificities. These strategies are based on "immortalization" of the immunoglobulin genes encoding the antibodies rather than the cell lines producing them.

For production of intact human monoclonal antibodies, scFv with desirable specificities can be inserted into mammalian expression vectors containing the genes encoding human immunoglobulin constant regions. We have recently developed a series of constructs that permit the rapid conversion of phage display library-derived scFv antibody fragments to fully human monoclonal antibodies of each immunoglobulin isotype and subclass (29,30). Transfected cell lines harboring these constructs produce human monoclonal antibodies *in vitro* that are correctly assembled and glycosylated 29,30). In a particularly preferred embodiment of the invention the binding molecule comprises a fully human antibody.

Another aspect the invention provides the use of a binding molecule of the invention for selectively marking a subset of CD38 positive cells. In another aspect the invention provides a method for marking a CD38-expressing cell with a binding molecule by contacting a collection of CD38 positive cells with a binding molecule capable of selectively binding to a subset of CD38 positive cells. Cells of the subset can be marked for diagnostic purposes. In such a case, the binding molecule preferably comprises a tag and/or a label through which the binding molecule can be directly or indirectly detected.

Marking can also serve to provide a signal for other proteins or cells to act upon marked cells, for instance for the binding of complement factors in the blood or the binding of (cytotoxic) T-cells. The marking may also serve to bring other essentially non-CD38 selective molecules in the vicinity and/or into the interior of the selected subset of CD38 positive cells. This property is useful in targeting approaches to carry material near and/or into the selected subset of cells. The material can vary from a toxic substance such as a radiolabel (for instance for killing cells of the selected subset) or a gene delivery vehicle (for the expression of a foreign gene in cells of the selected subset). Many forms of gene delivery have been described in the art and, depending on the gene delivered, different effects can be established. A suicide gene can be introduced to facilitate the killing of cells belonging to the subset, alternatively, genes capable of expressing immune stimulatory and/or inhibitory proteins can be introduced to modulate the immune activity of, for instance, marked T cells belonging to the subset of cells. It is clear to the person skilled in the art that combinations of marking uses can also be made. The marking can also be combined with the marking of cells through binding molecules with other specificities, to either broaden or further limiting the range of cells marked.

Another aspect the invention provides the use of a binding molecule of the invention for the preparation of a medicament. Administration of a binding molecule of the invention to an individual leads to the selective binding of the binding molecule to the selected subset of CD38 positive cells in said individual. Depending on the further properties of the binding molecule and/or associated material, the cell will perform a certain function or functions.

In a preferred embodiment, the medicament is used for the treatment of an individual suffering from a tumor of the lymphoid lineage, suffering from an autoimmune disease and/or suffering from an infection with a pathogen that depends, at least in part, on replication in activated T-cells and/or B-cells. Preferably, the binding molecule comprises a humanized or fully human antibody or a part, derivative and/or analogue thereof. Administration of the medicament to the individual will lead to activation of complement and/or the initiation of an immune response against the marked cells, resulting in cell death in at least part of the selected subset of cells. The selective cell death of the subset of CD38 positive cells leads at least to an improved clinical appearance of the individual suffering from the disease. In a preferred embodiment, the tumor of the lymphoid lineage comprises multiple myeloma.

In yet another aspect the invention provides an isolated and/or recombinant nucleic acid encoding a binding molecule of the invention. Such a nucleic acid is useful for providing a cell with the capacity to express a binding molecule of the invention. This property can be used to produce the binding molecule in preferably large quantities. The invention therefore also provides a cell expressing a CD38 binding molecule according to the invention.

With the knowledge of a selectively expressed CD38 epitope and the uses of such epitopes, it is entirely possible to find additional binding molecules capable of binding to said selectively expressed CD38 epitope. For instance, a cell expressing said selectively expressed CD38 epitope may be used to select antibodies capable of binding to said cell. In the art, many methods exist to find such antibodies. With the methods of the invention, it is possible to determine whether an identified antibody comprises a binding specificity for said selectively expressed epitope. Moreover, many methods exist in the art to eliminate antibodies not binding to the for instance CD38 or said selectively expressed epitope of CD38. For instance, one may pre-incubated an antibody with a CD38 expressing cell not expressing said selectively expressed CD38 epitope. The invention, therefore, further provides the use of a selectively expressed CD38 epitope for selecting a binding molecule to said epitope. Preferably, said selectively expressed epitope is present on a cell.

Anti-CD38 monoclonal antibodies may be divided into two broad subgroups, represented by the OKT10 and IB4 antibodies, that bind to non-overlapping epitopes (31). In antibody binding inhibition assays, binding of scFv UM16 to CD38 was almost completely blocked by the OKT10 and not by the IB4 monoclonal antibody. The epitope of the OKT10 antibody has been mapped to residues 280-298 at the carboxyl terminus of the 300 residue CD38 molecule, whereas the IB4 antibody has been mapped to residues 220-241 (32). These results suggest that at least part of the epitope of UM16 is located at the carboxyl terminus of CD38.

In immunoprecipitation and western blot analysis, the CD38 molecule could be precipitated from lysates of CD38+ cell lines, including CD38+ cell lines that did not expose the UM16 epitope as determined by membrane immunofluorescent staining. This observation suggests that on the plasma membrane of these cell lines, the UM16 epitope is shielded from interaction with scFv UM16 and that solubilization of the membranes during immunoprecipitation results in exposure of the UM16 epitope. The basis for shielding of the UM16 epitope is unclear. Since it has been shown that the CD38 molecule may be present on the membrane in different aggregation forms depending on cell lineage and or activation state, it may be envisaged that the exposure of UM16 is related to the degree of multimerization of CD38 (33-36). Alternatively, the UM16 epitope may be shielded as a result of lateral interactions of CD38 with other membrane molecules such as the B or T cell receptor, CD73 or PC-1 (37,38).

In sequential immunoprecipitation and western blot analysis of lysate of plasma cell line RPMI-8226, a second band of 30 kD molecular weight was detected with the IB4 antibody. This band was not observed in lysates of the Daudi or U266 cell lines. Alternative forms of CD38 at the mRNA and protein level have been reported (39-41). RNA encoding a splice variant of CD38 with a predicted molecular weight of 13,500 kD is present in almost all tissues and cell lines. A 39 kD soluble form of CD38 is present in biological fluids and supernatant of tumor cell lines (42,43). Furthermore, post-translational cross-linking has been shown to result in the expression of a 190 kD tetrameric CD38 complex on retinoic acid-treated HL-60 cells (33). The 30 kD form described here, detected with the IB4 but not with the OKT10 antibody, is compatible with a truncated CD38 molecule that lacks the carboxy terminus.

A number of monoclonal antibodies have been described which selectively recognize epitopes on activated or liganded integrins. Activation epitopes may arise as a result of ligand occupancy, interchain contactors between clustered integrins or other indirect mechanisms such as avidity shift upon activation (44-46). An example of the latter may be the epitope recognized by the NKI-L16 monoclonal antibody that recognizes an epitope on the LFA-1 integrin that is absent from resting lymphocytes and monocytes, but becomes exposed upon *in vitro* stimulation (47, 48). The NKI-L16 epitope is induced in the absence of upregulation of other LFA-1 epitopes, presumably as a result of a conformational change in LFA-1. In contrast, UM16 epitope detection coincided with upregulation of CD38 expression. The sheep red blood cell receptor-related T11₃ epitope described by Meuer *et. al* is expressed on activated but not resting T lymphocytes (49). Notably, an antibody against the non-overlapping T11₂ epitope rapidly induced expression of the T11₃ epitope within 30 minutes at 4 °C. Similarly, incubation of CD38++ Daudi cells with murine antibody IB4 which recognizes a non-overlapping CD38 epitope, inducing upregulation of the UM16 epitope within 20 minutes at 4 °C was noted. Thus, perturbation of the CD38 molecule by binding of monoclonal antibody appears to cause a conformational change or alteration in aggregation status of CD38, resulting in exposure of the UM16 epitope.

The functional consequence of UM16 expression by activated lymphocytes remains to be established. CD38 is a bi-functional ectoenzyme whose extracellular domain bears catalytic properties and is also involved in cell-cell interactions (11). CD38 has been implicated in proliferation, lymphopoiesis, apoptosis, adhesion and cytokine production (9, 50-55). CD31, a molecule expressed by a large number of cell types including monocytes and endothelial cells, has been shown to be a ligand for CD38 (37,56). Because *in vivo,* the UM16 epitope is expressed by a subpopulation of activated lymphocytes in primary and secondary lymphoid organs, we favor the possibility that the UM16 epitope is involved in cell-cell or cell-substrate interactions in those microenvironments. This notion is further supported by the observation that the UM16 epitope can be induced on BM-derived mononuclear cells, but not on blood B and T cells with mitogens. Apparently, the BM mononuclear cell fraction contains cells that by direct cell-cell contact or production of soluble molecules involved in the induction of this activation epitope. In that respect, it is noteworthy that within the family of integrin adhesion molecules, conformational changes and concomitant expression of neoepitopes leads to enhanced target binding (57). Similarly, expression of the UM16 epitope may be related to increased adhesive properties of CD38.

The recombinant scFv antibody UM16 appears to bind to activated B lineage cells, irrespective of their differentiation stage. This characteristic, in combination with the limited cellular distribution of the UM16 epitope, may render this human antibody fragment or derivatives thereof a suitable molecule to target tumors of the B lymphocyte lineage, including multiple myeloma. In addition, it is anticipated that UM16 may be used to target activated lymphocytes in autoimmune diseases such as rheumatoid arthritis and systemic lupus erytematosus.

### Examples

### Materials and Methods

### Patient materials

The human myeloma cell lines UM-2 was established from the pericardial effusion of a patient with end-stage myeloma. Continued proliferation of myeloma tumor cells was observed during culture with autologous BM-derived stroma cells. UM-2 phenotypical resembles a plasma cell line as determined by light microscopic evaluation of cytocentrifuge preparations stained with May-Grünwald-Giemsa and cell surface marker profile (described in detail in reference 58). Prior to use for phage selections, UM-2 cells were removed from the stroma layer by mechanical agitation.

### Tissue samples

Peripheral blood leukocytes were isolated from the blood of healthy volunteers by ficoll-hypaque density centrifugation or by lysis of erythrocytes by hypotonic NH₄Cl solution as described (4). Tonsils were obtained from children undergoing routine tonsillectomy, minced, squeezed through a nylon mesh filter and isolated by ficoll-hypaque density centrifugation. Fetal bones were collected from aborted human fetuses (14-20 weeks of gestation) and used according to the guidelines of the Utrecht University Hospital institutional review board on the use of human materials for scientific research. BM cells were obtained by flushing medullary cavities of the femurs with RPMI 1640 medium and ficoll density centrifugation. Adult BM aspirates were obtained with informed consent from healthy individuals undergoing allogeneic BM transplantation or coronary bypass surgery or from MM patients in stage III according to the criteria of Salmon and Durie (59). Details of the cell separation procedures have been described elsewhere (4).

### Cell lines

The following human cell lines were used in this study: the pro B cell lines TOM-1 (60) and BV173 (61), the pre B cell lines Reh (ATCC CRL-8286) and Nalm 6 (62), the mature B cell lines Bjab (kind gift from Dr. G. Moldenhauer) and Daudi (ATCC CCL-213), the T cell lines CEM (CCL-119), Jurkat (ATCC TIB-152) and H9 (HTB-176), the myeloid cell lines HL-60 (ATCC CCL-240) and U937 (ATCC CRL-1593), the plasmacytoid cell lines U266 (ATCC TIB 196), ARH-77 (CRL-1621), IM9 (CCL-159), Fravel (63), RPMI 8226 (ATCC CCL-155), HS-Sultan (ATCC CRL 1484), OPM-1 and OPM-2 (64), XG-1 (65), UM-1, UM-2, UM-3 and UM-6 were established at our own institute. All cell lines were grown in RPMI 1640 medium supplemented with 5 % fetal calf serum and antibiotics at 37 °C in a humidified atmosphere with 5 % CO₂.

### Selection and propagation of phages

Approximately 10 ¹³ recombinant antibody-bearing phage particles of a library of 4.10⁹ specifities cloned in vector pPvt were blocked for 15 min in 1 ml of PBS/4% milk powder. 6 x 10 ⁶ UM-2 cells were added to the blocked phages and the mixture was slowly rotated for 6 hours at 4 °C. Subsequently, non-bound phages were removed by washing the cells twice in 50 ml of ice- cold PBS/1% BSA. The pelleted cells were suspended in 100 µl of diluted phycoerytrin (PE)-conjugated CD38 antibody (Becton Dickinson, San Jose, CA). After 20 min of incubation on ice, cells were washed once with PBS/1%BSA and resuspended in 800 µl of ice-cold PBS/1% BSA. A total of 5x10⁴ cells UM-2 cells expressing high level of the CD38 molecule were sorted on a FACStar plus equipped with a neon laser (Becton Dickinson). Phages were eluted from the sorted cells by the addition of 150 µl of 76 mM citric acid (pH 2.5) in PBS followed by incubation for 5 min at room temperature. The mixture was immediately neutralized with 200 µl of 1M Tris-HCl pH 7.4. Eluted phages were used to infect *E. coli* XL1-Blue and the bacteria were plated on TYE medium containing the appropriate antibiotics and glucose. Bacterial colonies were counted, scraped from the plates, and used as inoculum for the next round of phage selection.

### Preparation of mΦAbs and immunofluorescence analysis

MΦAbs were prepared from individual colonies grown in 200 ml of 2TY medium, purified by polyethylene glycol precipitation, resuspended in 500 µl of PBS/1% BSA/0.1%NaN₃, filtered (0.45 µm), and stored at 4 °C (5). For immunofluorescence analysis, 15 µl of mΦAb was blocked by adding 50 µl of PBS/4% milk powder for 15 min at room temperature. Cells (5x10⁵) obtained from different tissues were resuspended in 20 µl of PBS/1%BSA, added to the pre-blocked phages and incubated on ice for 45 min. The cells were washed twice in ice-cold PBS/1%BSA, followed by incubation in 10 µl of a diluted (1:400) sheep anti M13 polyclonal antibody (Pharmacia, Uppsala, Sweden) for 30 min, washed once, and incubated in 10 µl of PE-labeled donkey anti-sheep polyclonal antibody solution (diluted 1:500) for 30 min. All incubations were carried out on ice. The cells were washed once and analyzed on a FACScan (Becton Dickinson, San Jose, CA). In double and triple staining experiments, mΦAb-labeled cells were incubated with 10 µl of tricolor-labeled CD38 monoclonal antibody (Monosan, Uden, The Netherlands) and either with FITC-labeled CD3, CD4, CD8, CD19, CD10, CD14, CD15, CD34, CD43 or CD44 monoclonal antibodies (all from Becton Dickinson) or with FITC-labeled CD77 or unconjugated CMRF-17 monoclonal antibodies (a kind gift of Drs J. Wiels and D.N.J. Hart respectively). In combinations with CMRF-17, a FITC-labeled goat anti-rat polyclonal antibody was used (Becton Dickinson). After a single final wash, the cells were resuspended in 0.5 ml of PBS/1%BSA and analyzed by flow cytometry using a FACScan.
The intensity of CD38 staining was arbitrarily classified as CD38+ (log fluorescence intensity between 1 and 2), CD38++ (log fluorescence intensity between 2 and 3) or CD38+++ (log fluorescence intensity higher than 3)

### Production of scFv antibodies

ScFv antibody fragments were produced in the *E. Coli* non-suppressor strain SF110, as described (5). The SF110 strain is deficient in the DegP and OmpT proteases resulting in the production of stable scFv fragments (66).
The DNA fragment encoding scFv UM16 was subcloned in the Notl/Ncol digested vector 1M3-HIS that adds 6 histidine residues (HIS-tag) to the carboxyl terminus of the scFv fragment. These constructs were introduced into *E. coli* strain SF110 and HIS-tagged scFvs were produced and purified over a nickel-charged column (Ni-NTA agarose, Qiagen) as described (67).

### COS-7 transfection and immunohistochemical staining

COS-7 cells were transfected with 2 µg of CDM8 vector containing the cDNA encoding the CD38 molecule using the DEAE-dextran/sulphate method. After 2 days of culture, cells were detached, washed with PBS, and incubated with scFv UM16 and either with control scFv or with FITC-labeled anti-CD38 (Immunotech), for 30 min on ice. The cells were washed twice with PBS/1%BSA and incubated with supernatant of a hybridoma cell line secreting the anti-myc peptide monoclonal antibody 9E10 (ATCC CRL 1729) for 30 min, followed by washing with PBS/1%BSA and incubation with FITC-labeled goat anti-mouse Ig antibody (Becton Dickinson). After a final wash cells were analyzed by FACScan.

### Immunoprecipitation and electrophoretic analysis

10 µg of purified scFv UM16-HIS were bound to Chelating Sepharose beads fast Flow (Pharmacia Biotech) following a modification of a previously described procedure (68). 2x10⁷ cells were washed twice with ice-cold PBS and lysed in lysis buffer (PBS containing 3% CHAPS and 1mM PMSF) at 4 °C for 30 min. The nuclei were removed by centrifugation (10 min 13000 rpm) and the lysates were precleared with chelating sepharose beads coated with a control HIS-tagged scFv antibody specific for thyroglobulin. The lysates were incubated with scFv UM16-HIS or a control His-tagged scFv and rotated for 3 hours at 4 °C . The Sepharose beads were collected by brief centrifugation and washed 6 times with ice-cold lysis buffer diluted 1:1 in HEPES buffer. Immunoprecipitated samples were dissolved in sample buffer and loaded in duplicate onto 10% SDS-PAGE gels, run and electro-transferred to nitrocellulose. Nitrocellulose membranes were incubated with IB4 or OKT10 anti-CD38 monoclonal antibodies followed by peroxidase-conjugated rabbit anti-mouse polyclonal Ig. Reactive bands were visualized by chemiluminescence (Boehringer Mannheim, Mannheim, Germany) and exposure to X-ray films.

### Antibody binding inhibition experiments

For binding inhibition experiments, scFv UM16 was labeled with FITC using standard procedures. 10⁵ Daudi cells were preincubated on ice with different concentrations of unconjugated anti-CD38 MAbs (IB4, IB6, SUN4B7, and OKT10) and subsequently incubated with FITC-labeled scFv UM16. Samples were washed with PBS/1% BSA/0.1% NaN3 and analyzed using a FACScan.

### In vitro culture

Ficoll-separated peripheral blood and adult BM mononuclear cells were seeded at a concentration of 2x10⁶ cells/ml in 48 wells microtiter plates and stimulated with phytohemagglutinin (PHA 5 ng/ml), phorbol myristic acid (PMA 3 ng/ml) or a combination of PMA and calcium ionophore (500 nM). After 2 days of stimulation, cells were harvested and stained with scFv UM16 in combination with fluorochrome-labeled CD3 or CD19 and tricolor-labeled CD38 monoclonal antibodies.

### Results

### Selection of mΦAbs

A large phage display library of human scFv antibodies was used in an attempt to select antibodies specific for malignant plasma cells from patients with multiple myeloma (MM). As a target for phage selections, we used the stroma cell-dependent plasma cell line UM-2, derived from the BM of a MM patient. After allowing the phages to bind to UM-2, non-bound phages were removed by washing, the cells stained with a PE-labeled anti-CD38 antibody, and plasma cells and attached phages were sorted based on high levels of CD38 expression (69). Subsequently, cell-bound phages were eluted and propagated for the second round of selection using the same approach. After the second round of selection, 55 individual bacterial colonies were picked and used for BStN1 fingerprint analysis. Five different fingerprints were observed and mΦAbs from clones representing each of these fingerprints were generated. In flowcytometric analysis, all five mΦAbs stained the UM-2 cell line (results not shown). Double-staining of tonsil, blood, fetal and adult BM mononuclear cells with the 5 mΦAbs in combination with fluor chrome-labeled CD38, CD19, CD3, CD34, CD14, CD4, CD10, CD43, CD8, CD44 and CD15 antibodies showed that mΦAb UM16 displayed a restricted distribution pattern (see next paragraph), whereas the other mΦAbs broadly stained cells of various hematopoietic lineages. MΦAb UM16 was selected for further analysis.

### The UM16 epitope is expressed on normal and malignant plasma cells.

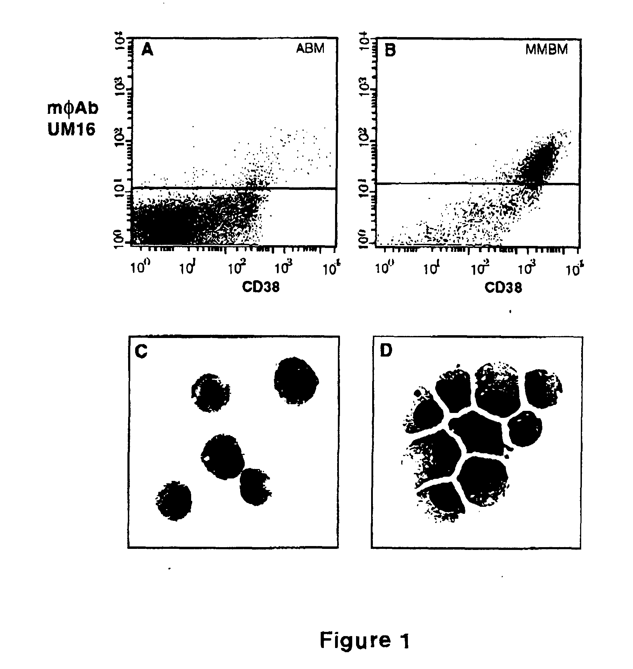

Adult BM mononuclear cells of healthy individuals (n = 8) and patients with MM (n = 8) were stained with mΦAb UM16 in combination with CD38. In BM samples of all healthy individuals, a small population of CD38+++/UM16+ cells could be detected with forward and side scatter properties characteristic of plasma cells (figure 1A). In all patients with MM, an expanded population of CD38+++ plasma cells stained with mΦAb UM16 (figure 1B). Cell sorting of UM16+ BM cells from a healthy donor and a patient with MM yielded a 90% pure population of cells with a characteristic plasma cell morphology (figure 1C and 1D) that showed bright cytoplasmic staining with anti-κ or anti-λ monoclonal antibodies (results not shown).

### Distribution of the UM16 epitope on freshly isolated hematopoietic cells

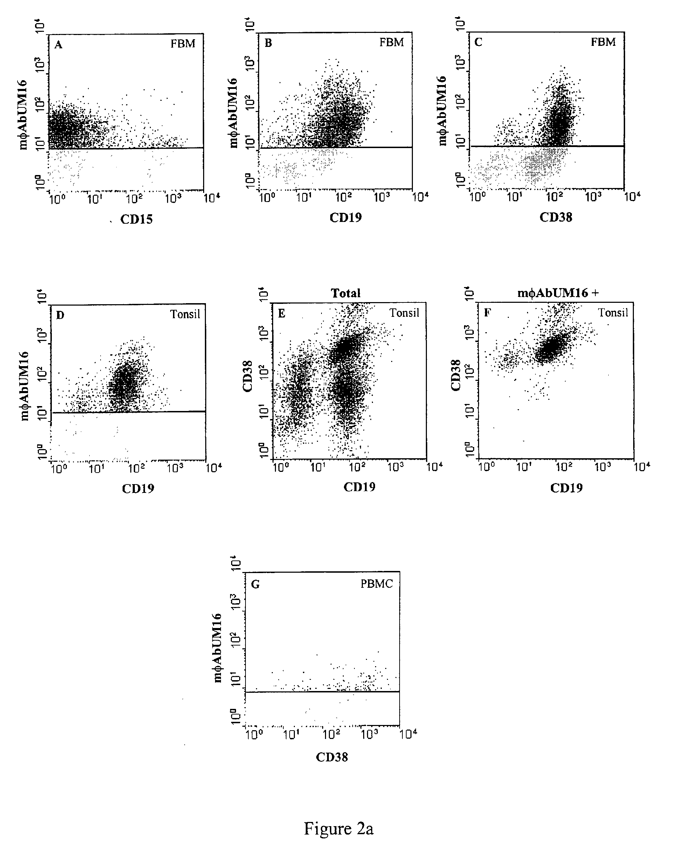

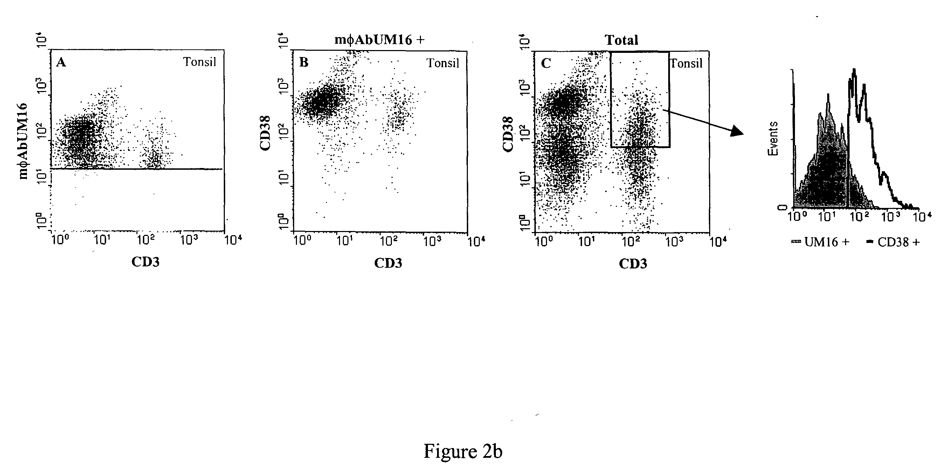

Mononuclear cells from fetal BM, tonsil and blood were double-stained with mΦAb UM16 and CD3, CD19, CD38 and CD15 antibodies and analyzed by flow cytometry. Representative staining patterns are shown in figure 2a and 2b.
In fetal BM, mΦAb UM16 did not stain CD15+ myeloid-lineage cells (figure 2a, A), whereas a large population of CD19+ B-lineage cells expressed the UM16 epitope (figure 2a, B). Double-staining with CD38 showed that the CD38++ but not the CD38+ cells expressed the UM16 epitope (figure 2a, C).
In tonsil, approximately 60% of the CD19+ B-lineage cells stained with mΦAb UM16 (figure 2a, D). Triple staining with CD38, CD19 and UM16 antibodies showed that all UM16+ cells expressed high (CD38++) or very high (CD38+++) levels of CD38 (Figure 2a, E and 2a, F). In blood < 0.5 % of the mononuclear cells expressed the UM16 epitope (fig 2a, G).
In tonsils, a subpopulation of CD3+ T cells was UM16+ in some tonsil samples analyzed (n=8; figure 2b, A ). Triple staining with CD38, CD3 and UM16 antibodies showed that all UM16+ cells represented a subset of the CD38++ T cells (figure 2b, B).

### Expression of the UM16 epitope during peripheral B cell maturation

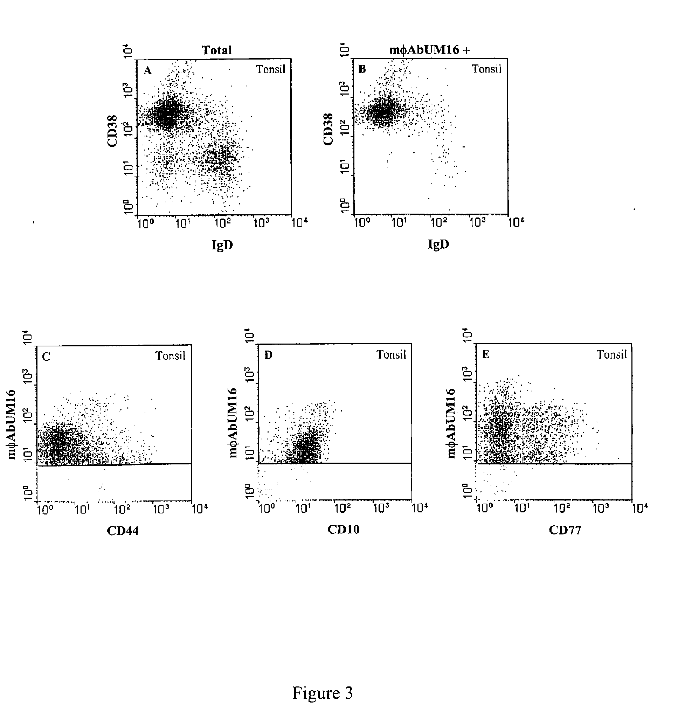

Using anti-IgD and CD38-specific monoclonal antibodies, human tonsillar B lymphocytes may be grouped into discrete subpopulations: IgD+/CD38- follicle mantle B cells, IgD-/CD38++ germinal center B cells, IgD-/CD38- memory B cells, IgD-/CD38++ plasmablasts and a small population of IgD+/CD38++ germinal center founder cells (70-74). To position UM16+ cells in this scheme of peripheral B cell maturation, triple staining of tonsillar B cells with mΦAb UM16 and fluorochrome-labeled anti-IgD and CD38 monoclonal antibodies was performed. The results showed that few if any IgD+/CD38- follicle mantle and IgD-/CD38- memory B cells stained with mΦAb UM16. In contrast, the IgD-/CD38++ germinal center B cells, the IgD-/CD38++ plasmablast and the IgD+/CD38++ germinal center founder B cell populations expressed the UM16 epitope (figure 3A and 3B). Further dissection of tonsillar B cell subpopulation using CD23, CD44 (75), CD77 (76), CD10 and CMRF17 (77) monoclonal antibodies in combination with mΦAb UM16 confirmed that UM16+ cells do not express the naive B cell markers CD23 (not shown) and CD44 (figure 3C). All UM16+ tonsil B cells expressed the germinal center markers CD10 (figure 3D) and CMRF17 (not shown), whereas a subpopulation of UM16+ cells expressed the CD77 marker expressed by germinal center centroblasts (figure 3E).

### Expression of the UM16 epitope during B cell differentiation in fetal BM.

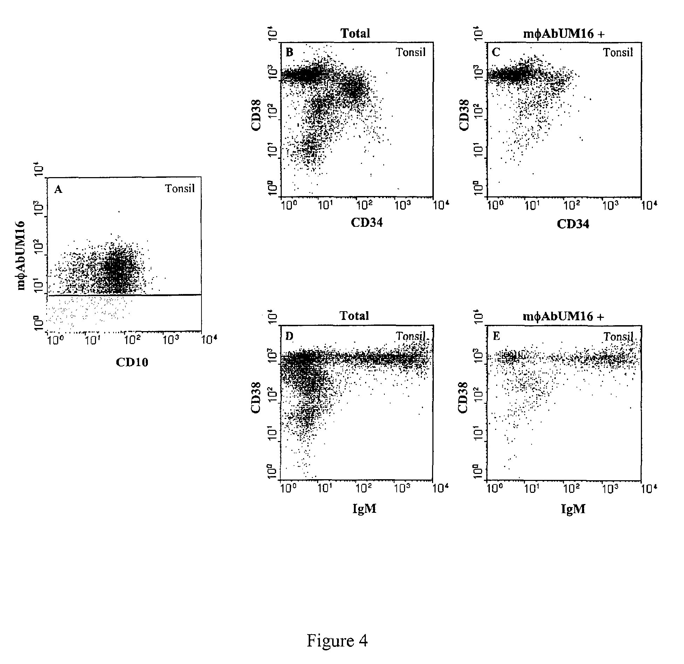

We noted that in human fetal BM, virtually all UM16+ cells expressed the B cell lineage-restricted CD19 antigen (figure 2B). In fetal BM, the earliest cells committed to the B-cell lineage express CD10, CD38 and intermediate levels of CD34 (12). As differentiation progresses, the CD34 and CD10 markers are lost and rearrangement and expression of immunoglobulin genes yields the more mature BM B-lineage cells. During these stages of fetal B cell differentiation, CD38 remains expressed on the cell surface (78). To position UM16+ cells in this scheme of early B cell differentiation, fetal BM cells were double-stained with mΦAb UM16 and CD10. Virtually all UM16+ cells expressed CD10, although a small population of CD10-/UM16+ cells could be discerned (figure 4A). In triple-staining with CD38, CD34 and UM16 antibodies, a small population of UM16+/CD34+/CD38++ and a large population of UM16+/CD34-/CD38++ was visible (figure 4B and 4C). Furthermore, triple staining of fetal BM cell with CD38, IgM and mΦAb UM16 unveiled UM16+/IgM^{dim}/CD38++ and UM16+/IgM^{bright} populations of B-lineage cells (figure 4D and 4E).

### Binding of mΦAb UM16 to CD38-transfected COS cells

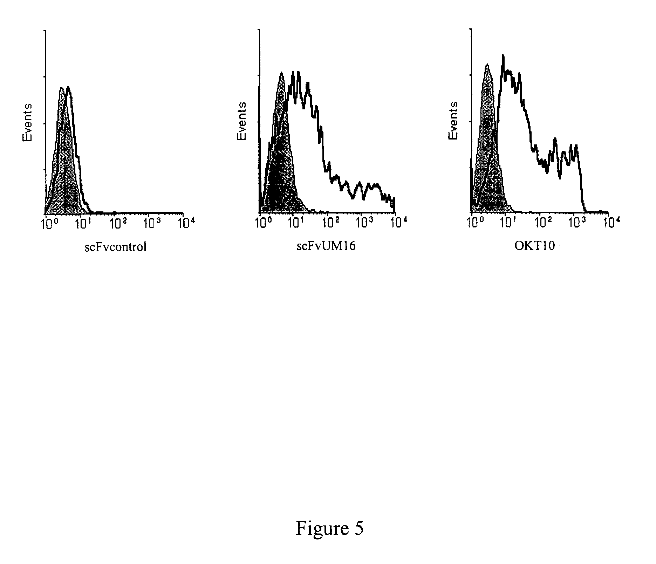

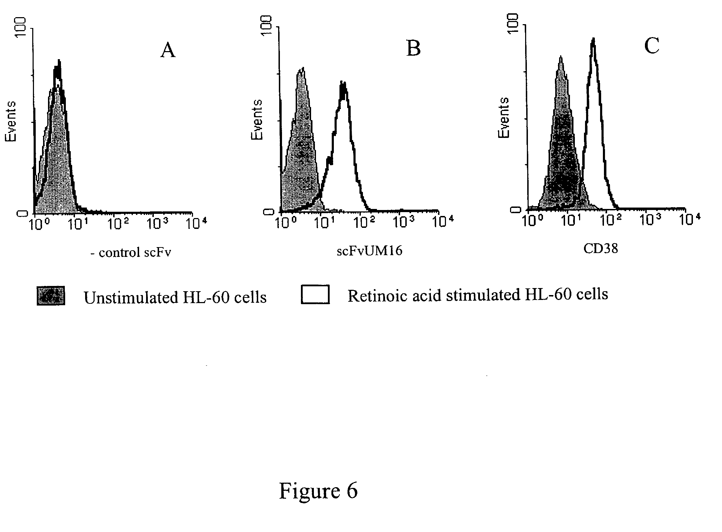

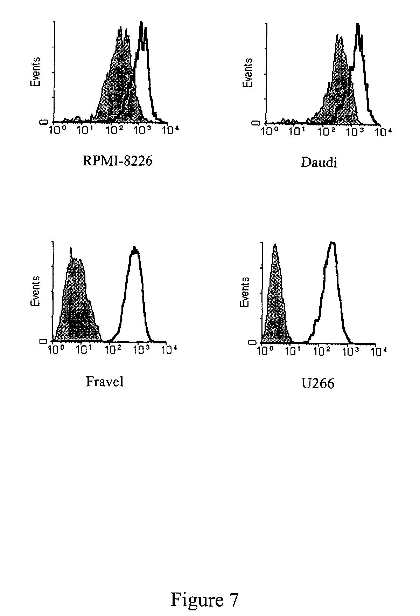

CD38 is a non cell lineage-restricted, multi-functional type II transmembrane glycoprotein broadly expressed on many cell types, including B and T lymphocytes, monocytes and NK cells (8,9,79). Although the UM16 epitope on human blood monocytes, T cells and NK cells was not detected, a partial overlap between the distribution of CD38 and the UM16 epitope was noted when viewed exclusively within the B cell lineage. This notion, in conjunction with the observation that, in the mouse, CD38 is expressed predominantly in normal and transformed B lymphocytes (80,81) prompted us to investigate whether the epitope detected by mΦAb UM16 was encoded by the CD38 gene. COS-7 cells were transfected with the full length cDNA encoding human CD38 and stained with scFv UM16 and a control scFv. Indeed, the CD38 transfected COS cells specifically stained with scFv UM16 and not with a control scFv antibody (figure 5).
To exclude the possibility that the differential staining patterns of conventional CD38 monoclonal antibodies and scFv UM16 could be attributed to differences in affinity, additional experiments were performed. Treatment of the erythroid leukemic cell line HL-60 with retinoic acid has been reported to result in the upregulation of CD38 expression (82-85). In the present experiments, treatment of HL-60 cells with retinoic acid resulted in up-regulation of CD38 expression and a concomitant upregulation of the UM16 epitope (figure 6). Prior to activation with retinoic acid, a low level of CD38 expression is detectable with the conventional anti-CD38 monoclonal antibody but not with scFv UM-16. After activation with retinoic acid, comparable levels of CD38 expression are detectable with both antibodies (figure 6). Further evidence of the differential expression of the UM16 epitope and other CD38 epitopes was obtained by screening a panel of CD38++ cell lines representing various hematopoietic lineages and differentiation stages. We noted that some cell lines expressed high levels of CD38, still lacked the UM16 epitope, in particular T lymphoid and myeloid cell lines (table 1). Representative staining patterns of four cell lines expressing comparable levels of CD38 yet differentially express the UM16 epitope are shown in figure 7. This observation corroborated the findings obtained using freshly isolated B and T lineage cells. In tonsil, fetal BM and BM from MM patients, CD38++ B-lineage cells uniformly exposed the UM16 epitope, whereas B lineage cells in the blood and BM of healthy individuals with comparable levels of CD38 expression failed to stain with scFv UM16 (compare figures 1A; 2a, C and 2a, G). In tonsil, CD38++ T cells could be UM16+ or UM16- (figure 2b, histogram).

### Inhibition of scFv UM16 binding to CD38 by anti-CD38 monoclonal antibodies

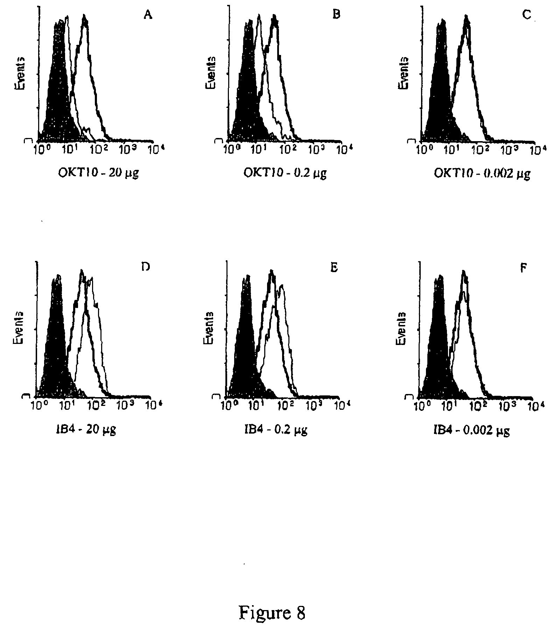

Based on epitope recognition patterns, murine anti-CD38 monoclonal antibodies may be divided into two broad subgroups. One subgroup is represented by OKT10 and SUN4B7 antibodies and recognize epitopes near the carboxyl-terminus, whereas the second subgroup detect a non-overlapping epitope of CD38 proximal to the transmembrane domain, as represented by the IB4 and IB6 antibodies. (31,32). To map the epitope detected by scFv UM16, we performed a binding inhibition assay using Daudi cells and OKT10, IB4 and FITC-labeled scFv UM16 antibodies. Results were monitored by flow cytometry. Preincubation of Daudi cells with monoclonal antibody OKT10 inhibited subsequent binding of FITC-labeled scFv UM16 (figure 8). Note that at concentrations as high as 0.2µg/ml and 20µg/ml, no complete inhibition was achieved (figure 8A and 8B). In contrast, preincubation of Daudi cells with monoclonal antibody IB4 at concentrations up to 20 µg/ml failed to inhibit subsequent binding of FITC-labeled scFv UM16. In fact, in four independent experiments we consistently observed an increase in UM16 expression as a result of binding of IB4, perhaps related to a perturbation of the CD38 molecule and associated increased accessibility of the UM16 epitope (figure 7D and 7E). This effect was not observed with the OKT10 antibody nor with a monoclonal antibody specific for CD19 (results not shown). Of note, the monosan anti-CD38 antibody used in all double staining experiments with scFv UM16 did not effect the levels of CD38 expression.

### The UM16 epitope is induced on in vitro activated T and B lymphocytes

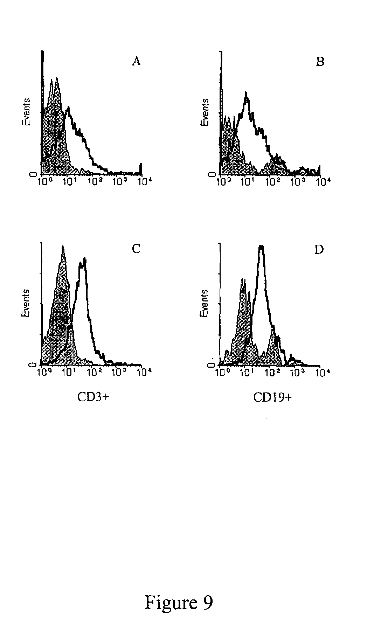

Activation of cells of lymphoid lineages is accompanied by increased expression of the CD38 molecule (10, 50). These results raised the possibility that mΦAb UM16 recognizes an epitope encoded by the CD38 molecule that can be induced on B lineage and T lineage cells upon activation.
To assess whether scFv UM16 recognized an activation epitope on B and T lymphocytes, adult BM and blood mononuclear cells were stimulated *in vitro* with PHA, PMA or a combination of PMA and calcium ionophore. After 2 days of culture, cells were harvested, stained with scFv UM16 and CD38 antibodies in combination with CD3 or CD19 antibodies and assayed by flow cytometry. Although we noted a clear increase in CD38 expression by mononuclear cells in blood and adult BM with all three stimulation regimen, expression of the UM16 epitope was induced on B and T lymphocytes in adult BM only (figure 9).

### Immunoprecipitation

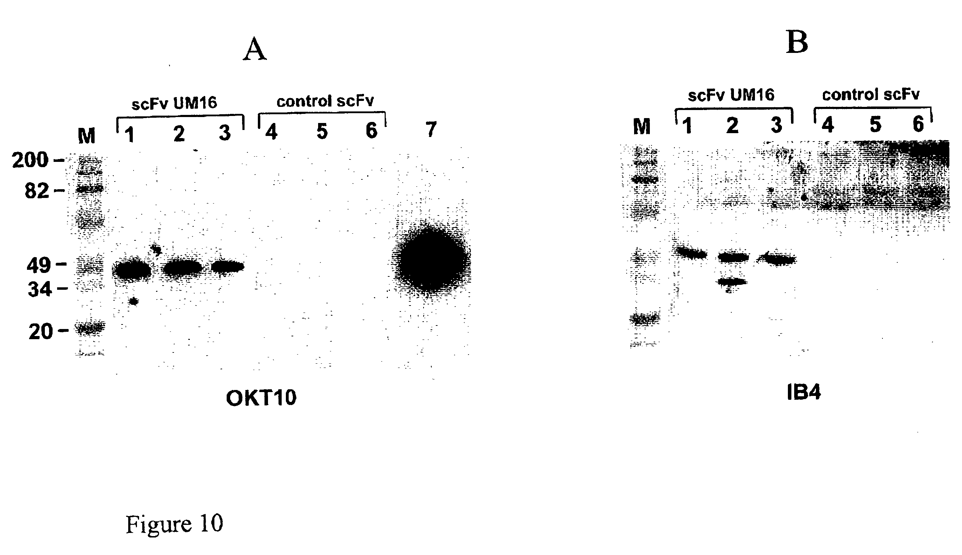

In view of the above results it is believed that the scFv UM16 detects an epitope on CD38 that is exposed upon activation of B and T lymphocytes. Thus the UM16 epitope should be detectable upon disruption of membranes of cells that are CD38+/UM16- by cell surface staining. Support for this hypothesis came from immunoprecipitation and western blotting analysis of a membrane lysate of the CD38+/UM16-plasma cell line U266. Lysates of U266, and the CD38+/UM16+ cell lines Daudi and RPMI-8226 as positive controls, were precipitated with scFv UM16 or a control anti-thyroglobulin scFv coupled to sepharose beads. The precipitates were run on SDS-PAGE, blotted to nitrocellulose and developed with the OKT10 or IB4 anti-CD38 monoclonal antibodies. The results of these experiment are shown in figure 10. No specific bands were detected after precipitation of lysates of all three cell lines with the negative control scFv (figure 10, lane 4,5,6). Precipitation of the U266, Daudi and RPMI-8266 lysates with scFv UM16 yielded a single band of approximately 46 kD molecular weight that ran at same height as control CD38 recombinant protein loaded on the same gel (figure 9, lane 7). It is noted that, in lysate of RPMI-8226 (figure 10, lane 2), western blot analysis with IB4 yielded a second band of approximately 30 kD, not visible in western blot analysis with the OKT10 monoclonal antibody.

### Brief description of the drawings.

### Figure 1.

Staining and cell sorting of cells expressing the UM16 epitope. Adult BM mononuclear cells from a healthy donor (panel A) or a patient with MM (panel B) was double-stained with CD38 and scFv UM16 antibodies and the UM16+ cells (black dots) were isolated by cell sorting and stained with May-Grünwald Giemsa (panel C and D)

### Figure 2a.

Staining patterns of hematopoietic cells from fetal BM and tonsil with mΦAb UM16. In panel A-D, G and H, UM16+ and UM16- cells are depicted as black and grey dots respectively. Panel A-C: fetal BM mononuclear cells double-stained with mΦAb UM16 and CD15-FITC, CD19-FITC or CD38-FITC. Panel D-F: tonsil mononuclear cells. Panel D: double-staining with mΦAb UM16 and CD19-FITC. Panel E and F, triple-staining with mΦAb UM16, CD38-tricolor and CD19-FITC. Panel E depicts the CD38/CD19 staining of the whole mononuclear cell fraction, whereas panel F depicts the position of the mΦAb UM16+ cells within the CD38/CD19 plot. Panel G: double-staining of peripheral blood mononuclear cells (PBMC) with mΦAb UM16 and CD38-FITC.

### Figure 2b.

Panel A Staining of tonsil mononuclear cells with CD3 and mΦAb UM16.
Panel B and C: tonsil mononuclear cells in triple-staining with mΦAb UM16-PE, CD38-tricolor and CD3-FITC. Panel B depicts the position of the mΦAb UM16+ cells within the CD38/CD3 plot. Panel C depicts the CD38/CD3 staining of tonsil cells whereas the histogram compares the expression of UM16 (grey histogram) and CD38 (thick solid line) within the gate drawn in panel C.

### Figure 3.

Expression of the UM16 epitope during peripheral B cell differentiation. Panel A-B: triple-staining of T cell-depleted tonsil mononuclear cells with mΦAb UM16, CD38-tricolor and anti-IgD-FITC. Panel A depicts the CD38/IgD staining whereas panel B depicts the position of UM16+ cells within the CD38/IgD plot. Panel C-E: double-staining of tonsil mononuclear cells with mΦAb UM16 and FITC-labeled CD44, CD10 and CD77 monoclonal antibodies.

### Figure 4.

Expression of the UM16 epitope during fetal BM B cell differentiation. Panel A: double-staining fetal BM mononuclear cells with mΦAb UM16 and CD10-FITC. Panel B-C: triple staining with mΦAb UM16, CD34 and CD38. Panel B depicts the CD38/CD34 staining of all mononuclear cells, whereas panel C depicts the position of UM16+ cells within the CD38/CD34 plot. Panel D-E: triple staining with mΦAb UM16, anti-IgM and CD38. Panel D depicts the CD38/IgM staining whereas panel E depicts the position of UM16+ cells within the CD38/IgM plot.

### Figure 5.

Staining of COS cells transfected with human CD38 cDNA with scFv UM16 and the tri-color labeled anti-CD38 monoclonal antibody. Grey histogram: staining with control scFv. Thick line: scFv UM16 or CD38 staining.

### Figure 6.

Staining of untreated (grey histogram) and retinoic acid-treated (transparent histogram) HL60 cells with a control scFv specific for thyroglobulin (panel A), scFv UM16 (panel B) and the tricolor-labeled murine anti-CD38 monoclonal antibody (panel C)

### Figure 7

Staining of RPMI-8226, Daudi, Fravel and U266 cell lines with scFv UM16 (grey histogram) and a murine monoclonal anti-CD38 antibody (thick line).

### Figure 8

Mapping of the UM16 epitope. Murine monoclonal anti-CD38 antibody OKT10 inhibits the binding of FITC-labeled scFv UM16 to Daudi cells at concentration of 20 µg/ml (panel A), and at 200 ng (panel B) but do effect at concentration of 2 ng/ml (panel C). No binding inhibition of FITC-labeled scFv UM16 to Daudi cells was observed with the monoclonal anti-CD38 antibody at IB4. Note an increase of binding of scFv UM16-FITC after incubation with the IB4 antibody at concentrations of 20 and 0.2 µg/ml but not 20 ng/ml (panel D and E).

Grey histogram: staining with the anti-thyroglobulin negative control scFv. Thick solid line: staining with scFv UM16-FITC. Thin solid line: staining with scFv UM16-FITC after binding of anti-CD38 (OKT10 or IB4) monoclonal antibodies.

### Figure 9

Expression of CD38 and UM16 on adult BM mononuclear cells stimulated with PMA and calcium ionophore. Histograms A and B represent staining with scFv UM16, whereas C and D depict staining with tricolor-labeled CD38. In panel A and C, CD3+ T cells are gated and in panel B and D CD19+ B cells are gated. Grey histograms: unstimulated cells. Thick solid line: PMA and Calcium ionophore stimulated cells.

### Figure 10

Immunoprecipitation and western blot analysis. Lysates of Daudi (lane 1 and 4), RPMI-8226 (lanes 2 and 5) and U266 (lane 3 and 6) cells were precipitated with beads coupled to scFv UM16 (lanes 1, 2, 3) or a control scFv specific for thyroglobulin (lanes 4, 5, 6). Precipitates were run on SDS-PAGE gel, blotted and the nitrocellulose filters were developed with the anti-CD38 MAbs OKT10 or IB4. Recombinant CD38 protein (lane 7) was loaded on the gel as a positive control.

Abbreviations: mΦAb, monoclonal phage antibody; scFv, single chain Fv; MM, multiple myeloma; BM, bone marrow; MAb, monoclonal antibody; PE, FITC, PMA.

### References

1. **Bodey, B., Bodey Jr, B., and Siegel, S.E.** Genetically-engineered monoclonal antibodies for direct anti-neoplastic treatment and cancer cell-specific delivery of chemotherapeutic agents. Curr. Pharm. Des. 2000. **27**:49-52.
2. **Winter, G., Griffiths, A. D., Hawkins, R. E. and Hoogenboom, H. R.,** Making antibodies by phage display technology. *Annu. Rev. Immunol.* 1994. **12:** 433-455.
3. **Burton, D. R. and Barbas III, C. F.,** Human antibodies from combinatorial libraries. *Adv. Immunol.* 1994. **57:** 191-280.
4. **de Kruif, J., Terstappen, L., Boel, E. and Logtenberg, T.,** Rapid selection of cell subpopulation-specific human monoclonal antibodies from a synthetic phage antibody library. *Proc. Natl. Acad. Sci. USA* 1995. **92:** 3938-3942.
5. **de Kruif, J., Boel, E. and Logtenberg, T.,** Selection and application of human single chain Fv antibody fragments from a semi-synthetic phage antibody display library with designed CDR3 regions. *J.Mol. Biol.* 1995. **248:** 97-105.
6. **van Ewijk, W., de Kruif, J., Germeraad, W. T. V., Berendes, P., Röpke, C., Platenburg, P. P. and Logtenberg, T.,** Subtractive isolation of phage-displayed single chain antibodies to thymic stromal cells using intact thymic fragments. *Proc. Natl. Acad. Sci. USA* 1997. **94:** 3903-3908.
7. **Hoogenboom**, **H. R. and Winter, G.,** By-passing immunisation. Human antibodies from synthetic repertoires of germline V_{H} gene segments rearranged *in vitro. J. Mol. Biol.* 1992. **227:** 381-388.
8. **Berthelier, V., Malavasi, F., Bismuth, G., Schmitt, C. and Deterre, P.,** Report on the 2nd international CD38 workshop. *Res. Immunol.* 1996. **147:** 407-411
9. **Mehta, K., Shahid, U. and Malavasi, F.,** Human CD38, a cell-surface protein with multiple functions. *FASEB J.* 1996. **10**: 1408-1417.
10. **Lund, F. E., Cockayne, D. A., Randall, T. D., Solvason, N., Schuber, F. and Howard, M. C.,** CD38: a new paradigm in lymphocyte activation and signal transduction. *Immunol. Rev.* 1998. **161:** 79-93.
11. **Shubinsky, G. and Schlesinger, M.,** The CD38 lymphocyte differentiation marker: new insights into its ecto-enzymatic activity and its role as a signal transducer. *Immunity* 1997. **7:** 315-324.
12. **Terstappen, L. W. M. M., Huang, S., Safford, M., Lansdorp, P. M. and Loken, M. R.,** Sequential generations of hematopoietic colonies derived form single non- lineage committed CD34+CD38- progenitor cells. *Blood* 1991. **77:** 1218-1227.
13. **Winter G., Griffiths, A.D., Hawkins, R.E., and Hoogenboom, H.R.** Making antibodies by phage display technology. Annu.Rev.Immunol. 1994. **12**:433-455.
14. **Griffiths, A.D., Willimas, S.C., Hartley, O., Tomlinson, I.M., Waterhouse, P., Crosby, W.L., Kontermann, R.E., Jones, P.T., Low, N.M., Allison, T.J. et al.** Isolation of high affinity human antibodies directly from large synthetic repertoires. EMBO J. 13:3245-3260.
15. **Shawler, D.L , Bartholomew, R.M., Smith, L.M. and Dillman, R.O.** Human immune response to multiple injections of murine monoclonal IgG. J. Immunol. 1985. **135**:1530-1535.
16. **Miller, R.A. Oseroff, A.R., Stratte, P.T. and Levy, R.** Monoclonal antibody therapeutic trials in seven patients with T-cell lymphoma. Blood 1983. **62:**988-995.
17. **Riechmann, L., Clark, M., Waldmann, H. and Winter, G.** Reshaping human antibodies for therapy. Nature 1988. **332**:323-327.
18. **Junghans, R.P., Waldmann, T.A., Landolfi, N.F., Avdalovic, N.M., Schneider, W.P. and Queen, C.** Anti-Tac-H, a humanized antibody to the interleukin 2 receptor with new features for immunotherapy in malignant and immune disorders. Cancer Res. 1990. **50**:1495-1502.
19. **Hakimi, J., Chizzonite, R., Luke, D.R., Familletti, P.C., Bailon, P., Kondas, J.A., Pilson, R.S., Lin, P., Weber, D.V., Spence, C., Mondini, L.J., Tsien, W.H., Levin, J.L., Gallati, V.H., Korn, L., Waldman, T.A., Queen, C. and Benjamin, W.R.** Reduced immunogenicity and improved pharmacokinetics of humanized anti-Tac in cynomolgus monkeys. J.Immunol. 1991. **147**:1352-1359.
20. **Stephens, S., Emtage, S., Vetterlein, O., Chaplin, L., Bebbington, C., Nesbitt, A., Sopwith, M., Athwal, D., Novak, C. and Bodmer, M.** Comprehensive pharmacokinetics of a humanized antibody and analysis of residual anti-idiotypic responses. Immunol. 1995. **85**:668-74. Immunology 85:668-674.
21. **Vaughan, T.J., Osbourn, J.K. and Tempest, P.R.** Human antibodies by design. Nat. Biotechnol. 1998. **16**:535-539.
22. **Khazaeli, M.B., Saleh, M.N., Liu, T.P., Meredith, R.F., Wheeler, R.H., Baker, T.S., et al.** Pharmacokinetics and immune response of 131I-chi-meric mouse/human B72.3 (human gamma4) monoclonal antibody in humans. Can-cer Res. 1989. **51**:5461-5466.
23. **Elliot, M.J., Maini, R.N., Feldman, M., Long-Fox, A., Charles, P., Bijl, H. et al.** Repeated therapy with monoclonal antibody to tumor necrosis factor alfa (cA2) in patients with rheumatoid arhritis. Lancet 1994. **344**:1125-1127.
24. **Jones, P.T., Dear, P.H., Foote, J., Neuberger, M.S. and Winter, G.** Replacing the complementarity-determining regions in a human antibody with those from a mouse. Nature 1986. **321**:522-525.
25. **Foote, J. and Winter, G.** Antibody framework residues affecting the the conformation of the hypervariable loops. J.Mol.Biol. 1992. **224**:487-499.
26. **Bruggeman, M. and Neuberger, M.S.** Strategies for expressing human antibody repertoires in transgenic mice. Immunol.Today 1996. **17**:391-397.
27. **Mendez, M.J., Green, L.L., Corvalan, J.R.F., Jia, X.-C., Maynard-Currie, C.E., Yang, X-d. et al.** Functional transplant of megabase human immunoglobulin loci recapitulates human antibody response in mice. Nat.Gen. 1997. **15**:146-156.
28. **Fishwild, D.M., O'Donnell, S.L., Bengoechea, T., Hudson, D.V., Harding, F., Bernhard, S.L., Jones, D., Kay, R.M., Higgins, K.M., Schramm, S.R., and Lonberg, N.** High-avidity human IgG kappa monoclonal antibodies form a novel strain of minilocus mice. Nat. Biotechnol. 1996. **14**:845-851.
29. **Abken, H., A. Hombach, U. Reinhold, S. Ferrone.** Can combined T-cell and antibody-based immunotherapy outsmart tumor cells? Immunol Today 1998. **19**:2.
30. **Maloney D.G., O.W. Press.** Newer treatments for non-Hodgkin's lymphoma: monoclonal antibodies. Oncology 1998. **12**:63.
31. **Morra, M., Deaglio, S., Mallone, R., Di Rosa, G., Tibaldi, E., Zini, G., Saccucci, F., Reinis, M., Horenstein, A. L., Funaro, A. And Malavasi, F.,** CD38 Workshop Panel report. Known B-cell CD antigens. *Leukocyte Typing VI* 1997, pp 151-154.
32. **Hoshino, S., kukimoto, I., Kontani, K., Inoue, S., Kanda, Y., Malavasi, F. and Katada, T.,** Mapping of the catalytic and epitopic stites of human CD38/NAD+ glycohydrolase to a functional domain in the carboxyl terminus. *J. Immunol.* 1997. **158:** 741-747.
33. **Umar, S., Malavasi, F. and Mehta, K.,** Post-translational modification of CD38 protein into a heigh molecular weight form alters it catalytic properties. *J. Biol. Chem.* 1996. **217:** 15922-15927.
34. **Franco, L., Zocchi, E., Calder, L., Guida, L., Benatti, U., De Flora, A.,** Self -aggregation of the transmembrane glycoprotein CD38 purified from human erythrocytes. ***Bioch. Bioph. Res. Com.*** 1994. 202: 1710-1715.
35. **Prasad, G. S., McRee, D. E., Stura, E. A., Levitt, D. G., Lee, H. C. and Stout, C. D.,** Crystal structure of Aplysia ADP ribosyl cyclase, a homologue of the bifunctional ectozyme CD38. *Nature Structural Biol.* 1996. **3** : 957-964.
36. **Ferrero, E. and Malavasi, F.,** The metamorphosis of a molecule: soluble enzyme to the leukocyte receptor CD38. *J. Leuk. Biol.* 1999. **65:** 151-161.
37. **Deaglio, S., Morra, M., Mallone, R., Ausiello, C. M., Prager, E., Garbarino, G., Dianzani, U., Stockinger, H., Malavasi, F.,** Human CD38 (ADP-ribosylcyclase) is a counter-receptor of CD31, an Ig superfamily member. *J. Immunol.* 1998. 160:395-402.
38. **Peola, S., Borrione, P., Matera, L., Malavasi, F., Pilieri, A. and Massaia, M.,** Selective induction of CD73 expression in human lymphocytes by CD38 ligation. A novel pathway linking signal transducers with ecto-enzyme activities. *J. Immunol.* 1996. **157**:4354-4362
39. **Jackson, D. G. and Bell, J.**, Isolation of a cDNA encoding the human CD38 (T10) molecule, a cell surface glycoprotein with an unusual discontinuous pattern of expression during lymphocyte differentiation. *J. Immunol.* 1989. **144:** 2811-2815.
40. **Nata, K., Takmura, T., Karasawa, T., Kunagai, T., Hashioka, W., Tohgo, A., Yonekura, H., Takasawa, S., Nakamura, S. and Okmota, H.**, Human gene encoding CD38 (ADP-ribosyl cyclase/cyclic ADP-ribose hydrolase):organization, nucleotide sequence and alternative splicing. *Gene* 1997. **186:** 285-292.
41. **Ferrero**, **E. and Malavasi, F.,** Human CD38, a leukocyte receptor and ectoenzyme, is a member of a novel eukariotic gene family od a nicotinamide adenosine dinucleotide⁺-converting enzymes. Extensive structural homology with the genes for murine bone marrow stromal cell antigen 1 and aplysian ADP-ribosyl cyclase. *J. Immunol.* 1997. **159:** 3858-3865.
42. **Funaro, A., Horenstein, A. L., Calosso, L., Morra, M., Tarocco, R. P., Franco, L., De Flora, A. and Malavasi, F.,** Identification and characterization of an active soluble form of human CD38 in normal and Pathological fluids. *Int. Immunol.* 1996. **8**:1643-1650.
43. **Mallone, R., Ferrua, S., Morra, M., Zocchi, E., Metha, K., Notorangelo, L. D. and Malavasi, F.,** Characterization of a CD38-like 78 kD soluble protein reased from B cell lines derived form patients with X-linked agammaglobulinemia. *J. Clin. Invest.* 1998. **101:** 2821-2830.
44. **Luque, A., Gomez, M., Puzon, W., Takada, Y., Sanchez-Madrid, F. and Cabanas, C.,** Activated conformations of very late activation integrins detected by a group of antibodies (HUTS) specific for a novel regulatory region (355-425) of the common beta 1 chain. *J. Biol. Chem.* 1996. **271:** 11067-11075.
45. **Elemer, G. S. and Edgington, T. S.,** Monoclonal antibody to an activation neoepitope of alpha M beta 2 inhibits multiple alpha M beta 2 functions. *J. Immunol.* 1994. **152:** 5836-5844.
46. **Boudignon-Proudhon, C., Patel, P. M. and Parise, L. V.,** Phorbol ester enhances integrin alpha IIb beta 3-dependent adhesion of human erythroleukemic cells to activation-dependent monoclonal antibodies. *Blood* 1996. **87:** 968-976.
47. **Morimoto, C., Rudd, C. E., Letvin, N. L., Schlossman, S. F.,** A novel epitope of the LFA-1 antigen which can distinguish killer effector and suppressor cells in human CD8 cells. *Nature* 1987. **330:** 479-482.
48. **van Kooyk, Y., Weder, P., Hogervorst, F., Verhoeven, A. J., van Seventer, G., te Velde, A. A., Borst, J., Keizer, G. D. and Figdor, C. G.,** Activation of LFA-1 through a Ca2+-dependent epitope stimulates lymphocyte adhesion. *J. Cell. Biol.* 1991. **112:** 345-354.
49. **Meuer, S. C., Hussey, R. E., Fabbi, M., Fox, D., Acuto, O., Fitzgerald, K. A., Hodgdon, J. C., Protentis, J.P., Schlossman, S.F. and Reinherz, E.L.,** An alternative pathway of T cell activation: a functional role for the 50 kd T11 sheep erythrocyte receptor protein. *Cell* 1984. **36:** 897-906.
50. **Funaro, A., Spagnoli, G. C., Ausiello, C. M., Alessio, M., Roggero, S., Delia, D., Zaccolo, M. and Malavasi, F.,** Involvement of the multilineage CD38 molecule in a unique pathway of cell activation and proliferation. *J. Immunol.* 1990. **145:** 2390-2396.
51. **Funaro, A., Morra, M., Calosso, L., Zini, M. G., Ausiello, C. M. and Malavasi, F.,** Role of the human CD38 molecule in B cell activation and proliferation. *Tissue Antigens.* 1997. **49:** 7-15
52. **Funaro, A., Reinis, M., Trubiani, O., Santi, S., Di primio, R. and Malavasi, F.,** CD38 functions are regulated through an internalization step. *J. Immunol.* 1998. **160:** 2238-2247.
53. **Kumagai, M., Coustan-Smith, E., Murray, D. J., Silvennoinen, O., Murti, K. G., Evans, W. E., Malavasi, F. and Campana, D.,** Ligation of CD38 suppresses human B lymphopoiesis. *J Exp Med* 1995. **181**:1101-1110.
54. **Zupo, S., Rugari, E., Dono, M., Taborelli, G., Malavasi,**
**F. and Ferrarini, M.,** CD38 signaling by agonistic monoclonal antibody prevents apoptosis of human germinal center B cells. *Eur J Immunol.* 1994. **24:** 1218-1222
55. **Ausiello, C. M., La Sala, A., Ramoni, C., Urbani, F., Funaro, A. and Malavasi, F.,** Secretion of IFN-gamma, IL-6, granulocyte-macrophage colony-stimulating factor and IL-10 cytokines after activation of human purified T lymphocytes upon CD38 ligation. *Cell. Immunol.* 1996. **173**:192-197.
56. **Cesano, A., Visonneau, S., Deaglio, S., Malavasi, F. and** **Santoli, D.,** Role of CD38 and its ligand in the regulation of MHC-nonrestricted cytotoxic T cells. *J. Immunol.* 1998. **160:** 1106-1115.
57. **Kolanus, W. and Seed, B.** Integrins and inside-out signal transduction:converging signals from PKC and PIP₃. *Curr. Opin. Cell biol.* 1997. **9:** 725-731.
58. **Bloem, A. C., Lamme, T., de Smet, M., Kok, H., Vooijs, W., Wijdenes, J., Boom, S. E. and Lokhorst, H.,** Long-term bone marrow cultured stromal cells regulateMyeloma tumour growth: in vitro studies with primary tumour cells and LTBMC-dependent cell lines. *Br. J. Haematol.*1998. **100** :166-175.
59. **Durie, B. G. M. and Salmon, S. E.,** A clinical staging system for multiple myeloma. Correlation of measured myeloma cell mass with presenting clinical features, response to treatment and survival. *Cancer.* 1975. **36**:842-854.
60. **Okabe, M., Matsushima, S., Morioka, M., Kobayashi, M., Abe, S., Sakurada, K., Kakinuma, M. and Miyazaki, T.,** Establishment and characterization of a cell line TOM-1, derived from a patient with Philadelphia chromosome-positive acute lymphocytic leukemia. *Blood* 1987. **69**:990-998
61. **Pegoraro, L., Matera, L., Ritz, J., Levis, A., Palumbo, A. and Biagnini, G.,** Establishment of a Ph1-positive human cell line (BV173). *J. Natl. Cancer* Inst.1983. **70**:447-453
62. **Hurwitz, R., Hozier, J., LeBien, T., Minowada, J., Gajl-Peczalska, K., Kubonishi, K. and Kersey, J.,** Characterization of a leukemic cell line of the pre-B phenotype. *Int J Cancer* 1979. **23:** 174-180
63. **Miller, C. H., Carbonell, A., Peng, R., Paglieroni, T. and Mackenzie, M. R.,** A human plasma cell line. Induction and characterization. *Cancer* 1982. **49:** 2091-2096.
64. **Katagiri, S., Yonezawa, T., Kuyama, J., Kanayama, Y., Nishida, K., Abe, T., Tamaki, T., Ohnishi, M. and Tarui, S.,** Two distinct human myeloma cell lines originating from one patient with myeloma. *Int. J. Cancer* 1985. **36:** 241-246.
65. **Zhang, X-G., Gaillard, J.P., Robillard, N., Lu, Z-Y., Gu, Z-J., Jourdan, M., Boiron, J.M., Bataille, R. and Klein, B.,** Reproducible obtaining of human Myeloma cell lines as a model for tumour stem cell study in human Multiple Myeloma. *Blood* 1994. *83:* 3654-3663.
66. **de Kruif, J. and Logtenberg, T.,** Leucine-zipper dimerized bivalent and bispecific antibodies from a semi-synthetic antibody phage display library. *J. Biol. Chem*.1996. **271:** 7630-7634.
67. **de Kruif, J., Storm, G., van Blois, L. and Logtenberg,** T., Biosynthetically lipid-modified human scFv fragments from a phage display library as targeting molecules for immunoliposomes. *FEBS lett.1996.* **399:** 232-236
68. **Hale, J. E.,** Irreversible, oriented immobilization of antibodies to cobalt-iminodiacetate resin for use as immunoaffinity media. *Anal. Biochem.* 1995. **231**:46-49
69. **Yi, Q., Dabadghao, S., Osterborg, A., Bergenbrant, S. and Holm, G.,** Myeloma Bone Marrow plasma cells: evidence for their capacity as antigen-presenting cells. *Blood* 1997. **90**:1960-1967
70. **Lagresle, C., Bella, C. and Defrance, T.,** Phenotypic and functional heterogeneity of the IgD- B cell compartment: identification of two major tonsillar B cell subsets. *Int. Immunol.* 1993. **5**:1259-1268.
71. **Billian, G., Bella, C., Mondiere, P. and Defrance, T.,** Identification of a tonsil IgD⁺ B cell subset with phenotypical and functional characteristics of germinal centre B cells. *Eur. J. Immunol. 1996.* **26**:1712-1719.
72. **MacLennan, I. C. M.**, Germinal centres. *Annu. Rev. Immunol.* 1994. **12**:117-139.
73. **Liu, Y. J. and Arpin, C.,** Germinal centre development. *Immunol. Rev.* 1997.**156**: 111-126. 74. **Liu, Y. J., Malisan, F., de-Bouteiller, O., Guret, C., Lebecque, S., Banchereau, J., Mills, F. C., Max, E. E. and** **Martinez-Valdez, H.,** Within germinal centres, isotype switching of immunoglobulin genes occurs after the onset of somatic mutation. *Immunity* 1996. **4:** 241-250.
75. **Kremmidiotis, G. and Zola, H.,** Changes in CD44 expression during B cell differentiation in the human tonsil. *Cellular Immunol.* 1995. **161:**147-157.
76. **Wiels, J.,** CD77 Workshop Panel report. Known B-cell antigens. *Leucocyte Typing VI* 1997, pp 175-177.
77. **Peach, S. F., Davidson, S. E., McKenzie, J. L., Nimmo, J. C. and Hart, D. N. J.,** An activation antigen on a subpopulation of B lymphocytes identified by the monoclonal antibody CMRF-17. *Immunol.* 1989. **67:** 351-358.
78. **Le Bien, T., Wormann, B., Villablanca, J. D., Law, L. M., Steinber, L. M., Shah, V. O. and Loken, M. R.,** Multiparameter flowcytometric analysis of fetal bone marrow B cells. *Leukemia* 1990. **4:** 354-358.
79. **Alessio, M., Roggero, S., Funaro, A., De Monte, L. B., Peruzzi, L., Geuna, M. and Malavasi, F.,** CD38 molecule: structural and biochemical analysis on human T lymphocytes, thymocytes and plasma cells. *J. Immunol.* 1990. **145:**878-884.
80. **Lund, F., Solvason, N., Grimaldi, J. C., Parkhouse, M. E. and Howard, M.,** Murine CD38: an immunoregulatory ectoenzyme. *Immunol. Today* 1995. **16: 469-473.**
81. **Oliver, A. M., Martin, F. and Kearney, J. F.,** Mouse CD38 is down-regulated on germinal centre B cells and mature plasma cells. *J. Immunol.* 1997. **158**: 1108-1115.
82. **Mehta, K., McQueen, T., Manshouri, T., Andreeff, M., Collins, S. and Albitar, M.,** Involvement of retinoic acid receptor-α-mediated signaling pathway in induction of CD38 cell-surface antigen. *Blood* 1997. **89:** 3607-3614.
83. **Drach, J., Zhao, S., Malavasi, F. and Mehta, K.,** Rapid induction of CD38 on myeloid leukemia cells by retinoic acid. *Biochem. Biophys. Res. Commun.* 1993. **195:** 545-550.
84. **Drach, J., McQueen, T., Engel, H., Andreeff, M., Robertson, K. A., Collins, S. J., Malavasi, F. and Mehta, K.,** Retinoic acid-induced expression of CD38 antigen in myeloid cells is mediated through retinoic acid receptor-alpha. *Cancer Res.*1994. **54:** 1746-1752.
85. **Kontani, K., Kukimoto, I., Kanda, Y., Inoue, S-i., Kishimoto, H., Hoshino, S-i. and Katada, T.,** Induction of CD38/NADase and its monoclonal antibody-induced tyrosine phosphorylation in human leukemia cell lines. *Biochem. Biophys. Res. Commun.* 1996. **222:** 466-471.

## Claims

1. A binding molecule capable of selectively binding to a subset of CD38 proteins.

2. A binding molecule according to claim 1, capable of selectively binding to a subset of CD38 protein expressing cells.

3. A binding molecule according to claim 2, wherein said subset of cells comprises a plasma cell and/or a derivative thereof.

4. A binding molecule according to claim 2 or claim 3, wherein said subset of cells comprises an activated hemopoietic cell.

5. A binding molecule according to claim 4, wherein said subset of cells comprises an in vivo activated hemopoietic cell.

6. A binding molecule according to anyone of claims 2-5, wherein said subset of cells comprises a T-cell and/or B-cell.

7. A binding molecule according to anyone of claims 1-6, wherein said binding molecule and/or a CD38 binding part thereof is selected from a phage display library.

8. A binding molecule according to claim 7, wherein said binding molecule comprises a CD38 binding part of a single chain antibody.

9. A binding molecule according to anyone of claims 1-8, wherein said molecule comprises a human antibody and/or humanized Fab-fragment or a functional part, derivative and/or analogue thereof.

10. Use of a binding molecule according to anyone of claims 1-9 for selectively marking a subset of CD38 positive cells.

11. Use of a binding molecule according to anyone of claims 1-9 for the preparation of a medicament.

12. Use of a binding molecule according to anyone of claims 1-9 for the treatment of an individual suffering from a tumor of the lymphoid lineage, suffering from an autoimmune disease and/or suffering from an infection with a pathogen that depends at least in part on replication in activated T-cells and/or B-cells.

13. Use according to claim 12, wherein said tumor of the lymphoid lineage comprises multiple myeloma.

14. A method for marking a CD38 expressing cell with a binding molecule comprising contacting a collection of CD38 positive cells with a binding molecule capable of selectively binding to a subset CD38 positive cells.

15. An isolated and/or recombinant nucleic acid encoding a binding molecule according to anyone of claims 1-9.

16. A cell expressing a CD38 binding molecule according to anyone of claims 1-9.

17. Use of a selectively expressed CD38 epitope for selecting a binding molecule to said epitope.
